# EUROPEAN PATENT APPLICATION

(11) **EP 2 354 244 A1**
(43) Date of publication of application: **10.08.2011**
(21) Application number: 10152654.9
(22) Date of filing: 04.02.2010
(51) Int. Cl.: C12Q 1/68, G01N 33/564

(54) **MBL2 as a marker of liver-specific insulin resistance**

(71) Applicant: Stichting Top Institute Food and Nutrition, 6709 PA Wageningen (NL)
(72) Inventor: Rakhshandehroo, Maryam, 6706 GA Wageningen (NL); Kersten, Alexander Henricus, 6078 KK Wageningen (NL); Müller, Michael, 6721 SG Bennekom (NL)
(74) Representative: Swinkels, Bart Willem

(57) **Abstract**

The invention relates to a use of a MBL2 as a biomarker of liver-specific insulin resistance and to a diagnostic method wherein said MBL2 is being used.

SEQUENCE LISTING

<110>  Stichting Top Institute Food and Nutrition

<120>  MBL2 as a marker of liver specific insulin resistance

<130>  P6029660EP

<160>  4

<170>  PatentIn version 3.3

<210>  1
<211>  3569
<212>  DNA
<213>  homo sapiens

<400>  1
ggtaaatatg tgttcattaa ctgagattaa ccttccctga gttttctcac accaaggtga      60

ggaccatgtc cctgtttcca tcactccctc tccttctcct gagtatggtg gcagcgtctt     120

actcagaaac tgtgacctgt gaggatgccc aaaagacctg ccctgcagtg attgcctgta     180

gctctccagg catcaacggc ttcccaggca aagatgggcg tgatggcacc aagggagaaa     240

agggggaacc aggccaaggg ctcagaggct tacagggccc ccctggaaag ttggggcctc     300

caggaaatcc agggccttct gggtcaccag gaccaaaggg ccaaaaagga gaccctggaa     360

aaagtccgga tggtgatagt agcctggctg cctcagaaag aaaagctctg caaacagaaa     420

tggcacgtat caaaaagtgg ctcaccttct ctctgggcaa acaagttggg aacaagttct     480

tcctgaccaa tggtgaaata atgacctttg aaaaagtgaa ggccttgtgt gtcaagttcc     540

aggcctctgt ggccaccccc aggaatgctg cagagaatgg agccattcag aatctcatca     600

aggaggaagc cttcctgggc atcactgatg agaagacaga agggcagttt gtggatctga     660

caggaaatag actgacctac acaaactgga acgagggtga acccaacaat gctggttctg     720

atgaagattg tgtattgcta ctgaaaaatg gccagtggaa tgacgtcccc tgctccacct     780

cccatctggc cgtctgtgag ttccctatct gaagggtcat atcactcagg ccctccttgt     840

ctttttactg caacccacag gcccacagta tgcttgaaaa gataaattat atcaatttcc     900

tcatatccag tattgttcct tttgtgggca atcactaaaa atgatcacta acagcaccaa     960

caaagcaata atagtagtag tagtagttag cagcagcagt agtagtcatg ctaattatat    1020

aatattttta atatatacta tgaggcccta tcttttgcat cctacattaa ttatctagtt    1080

taattaatct gtaatgcttt cgatagtgtt aacttgctgc agtatgaaaa taagacggat    1140

ttatttttcc atttacaaca aacacctgtg ctctgttgag ccttcctttc tgtttgggta    1200

gagggctccc ctaatgacat caccacagtt taataccaca gctttttacc aagtttcagg    1260

tattaagaaa atctattttg taactttctc tatgaactct gttttctttc taatgagata    1320

ttaaaccatg taaagaacat aaataacaaa tctcaagcaa acagcttcac aaattctcac    1380

acacatacat acctatatac tcactttcta gattaagata tgggacattt ttgactccct    1440

agaagccccg ttataactcc tcctagtact aactcctagg aaaatactat tctgacctcc    1500

atgactgcac agtaatttcg tctgtttata aacattgtat agttggaatc atattgtgtg    1560

taatgttgta tgtcttgttt actcagaatt aagtctgtga gattcattca tgtcatgtgt 1620
acaaaagttt catccttttc attgccatgt agggttccct tatattaata ttcctcagtt 1680
catccattct attgttaata ggcacttaag tggcttccaa tttttggcca tgaggaagag 1740
aacccacgaa cattcctgga cttgtctttt ggtggacatg gtgcactaat ttcactacct 1800
atccaggagt ggaactggta gaggatgagg aaagcatgta ttcagcttta gtagatatta 1860
ccagttttcc taagtgattg tatgaattta tgctcctacc ggcaatgtgt ggcagtccta 1920
gatgctctat gtgcttgtaa aaagtcaatg ttttcagttc tcttgatttt cattattcct 1980
gtggatgtaa agtgatattt ccccatggtt ttaatctgta tttccccaac atgtaataag 2040
gttgaacact tttttatatg cttattgggc acttgggtat cttcttttgt gaagtacccg 2100
ttcacatttt tgtattttgt ttaaattagt tagccaatat ttttcttact gatttttaag 2160
ttatttttac attctgaata tgtccttttt aatgtgtatt acaaatattt tgctagtttt 2220
tgacttgctc ctaatgttga attttgatga acaaaatttc ctaattttga gaaagtctta 2280
tttattcata ttttctttca aaattagtgc tttttgtgtc atgtttaaga aatttttgcc 2340
catcccaaaa tcataagata tttttcatga ttttgaaacc atgaagagat ttttcatgat 2400
tttgaaatca tgaagatatt ttttccattt tttctaatag ttttattaat aaacattcta 2460
tctattcctg gtagaataga tatccacttg agacagcact atgtaggaaa gaccattttt 2520
cctccactga actagggtgg tgcatttttg taagttaggt aactgtatgt gtgtgtgtct 2580
gtttctgggc tgtctattct agtctatttg ttgatgcttg tgtcaaacag tacactatct 2640
taattattgt acatttatag ttgtaactat agtccagctt tgttcttctt aaagtcaaga 2700
tttccatata aatattagaa acagcttctc aatttctaca aaatcctgat gaggtttcta 2760
ctgggaccac attgagtcta tcaatcaact tatgcagaac tggcaactta ctactgaatc 2820
tctaatcaat gttcatcatg tatcgcttca tgtaactaga atttctttaa cttaattgct 2880
atgttttgac atttttagtt taaaaacctt gtatatcttg ttttggtggt tttagtgatt 2940
ttaataatat attttaaata ttttttcttt tctattgttg tacacagaaa tacagttaag 3000
ttttgtgtgt agtcttacga tgtttagtaa actcaataag tttatttctt aaatctagta 3060
atttgtagat tcctctggat tttgtatatg catagtcatg taagctgaaa atatggcaat 3120
acttgcttct tcccaattgc tttacctttt ttcttacctt attgcactgg ttagcaaccc 3180
caatacagag accaccagat caggtataga ctcctgaaag acaatataat gaagtgctcc 3240
agtcaggcct atctaaactg gattcacagc tctgtcactt aattgctaca tgatctagag 3300
ccagttactt tgtgtttcag ccatgtattt gcagctgaga gaaaataatc attcttattt 3360
catgaaaatt gtggggatga tgaaataagt taacaccttt aaagtgtgta gtaaagtatc 3420
aggatactat attttaggtc ttaatacaca cagttatgcc gctagataca tgctttttaa 3480
tgagataatg tgatattata cataacacat atcgattttt aaaaattaaa tcaaccttgc 3540
tttgatggaa taaactccat ttagtcaca 3569

<210>  2
<211>  248
<212>  PRT
<213>  homo sapiens

<400>  2

Met Ser Leu Phe Pro Ser Leu Pro Leu Leu Leu Leu Ser Met Val Ala
1               5                   10                  15

Ala Ser Tyr Ser Glu Thr Val Thr Cys Glu Asp Ala Gln Lys Thr Cys
            20                  25                  30

Pro Ala Val Ile Ala Cys Ser Ser Pro Gly Ile Asn Gly Phe Pro Gly
        35                  40                  45

Lys Asp Gly Arg Asp Gly Thr Lys Gly Glu Lys Gly Glu Pro Gly Gln
    50                  55                  60

Gly Leu Arg Gly Leu Gln Gly Pro Pro Gly Lys Leu Gly Pro Pro Gly
65                  70                  75                  80

Asn Pro Gly Pro Ser Gly Ser Pro Gly Pro Lys Gly Gln Lys Gly Asp
                85                  90                  95

Pro Gly Lys Ser Pro Asp Gly Asp Ser Ser Leu Ala Ala Ser Glu Arg
            100                 105                 110

Lys Ala Leu Gln Thr Glu Met Ala Arg Ile Lys Lys Trp Leu Thr Phe
        115                 120                 125

Ser Leu Gly Lys Gln Val Gly Asn Lys Phe Phe Leu Thr Asn Gly Glu
    130                 135                 140

Ile Met Thr Phe Glu Lys Val Lys Ala Leu Cys Val Lys Phe Gln Ala
145                 150                 155                 160

Ser Val Ala Thr Pro Arg Asn Ala Ala Glu Asn Gly Ala Ile Gln Asn
                165                 170                 175

Leu Ile Lys Glu Glu Ala Phe Leu Gly Ile Thr Asp Glu Lys Thr Glu
            180                 185                 190

Gly Gln Phe Val Asp Leu Thr Gly Asn Arg Leu Thr Tyr Thr Asn Trp
        195                 200                 205

Asn Glu Gly Glu Pro Asn Asn Ala Gly Ser Asp Glu Asp Cys Val Leu
    210                 215                 220

Leu Leu Lys Asn Gly Gln Trp Asn Asp Val Pro Cys Ser Thr Ser His
225                 230                 235                 240

Leu Ala Val Cys Glu Phe Pro Ile

                245

<210> 3
<211> 22
<212> DNA
<213> artificial

<220>
<223> primer

<400> 3
gcaaacagaa atggcacgta tc 22

<210> 4
<211> 21
<212> DNA
<213> artificial

<220>
<223> primer

<400> 4
ctggaacttg acacacaagg c 21

## Description

### Field of the invention

The invention relates to a use of a MBL2 (Mannose-binding lectin or Mannose binding protein) as a biomarker of liver-specific insulin resistance and to a method wherein said MBL2 is being used.

### Background of the invention

The liver is a key organ in the control of lipid metabolism and whole energy homeostasis. Although not generally recognized as an important contributor to endocrine regulation of energy metabolism, recent studies point to the liver as a major source of secreted proteins that have profound metabolic effects elsewhere in the body [1]. Studies in mice have demonstrated that many aspects of hepatic lipid metabolism are under transcriptional control of the Peroxisome Proliferator Activated Receptor α (PPARa), a transcription factor belonging to the nuclear receptor superfamily . Lack of PPARα in mice leads to acute energy shortage in liver upon fasting and is characterized by defective ketone body formation, hypoglycemia, elevated plasma free fatty acids, and severe hepatic steatosis [2, 3]. Recently, it has been shown that PPARα also governs the hepatic production of FGF21 and ANGPTL4 [4, 5] which represent secreted proteins that majorly impact lipid metabolism. While FGF21 has been shown to serve as a mediator of the PPARa-induced starvation response in mice [6, 7] ANGPTL4 is now well established as a potent regulator of plasma triglyceride levels via inhibition of lipoprotein lipase [8]. The present study was undertaken to identify potential novel circulating mediators of PPARα activity in human.

Due to the growing importance of diabetes and/or liver-specific insulin resistance in western societies, there is a great need for specific markers that could be used in a method for diagnosing such disease or condition in a subject. Such markers are yet not available.

### Description of the invention

The invention provides the insight that a MBL2 gene may be used as a biomarker of liver-specific insulin resistance.

In a first aspect there is provided the use of a MBL2 as a biomarker of liver-specific insulin resistance. Within the context of the invention, a MBL2 may be replaced by a MBL2 gene or a MBL2 nucleic acid or a MBL2 encoded polypeptide.

In a second related aspect, there is provided a method for diagnosing liver-specific insulin resistance in a subject, the method comprising the steps of:
(a) determining the expression level of a MBL2 gene represented by a nucleic acid sequence selected having at least 60% identity with SEQ ID N0:1 in a subject; and,
(b) comparing the expression level of said gene as defined in (a) with a reference value for said expression level, the reference value preferably being the average value for said expression level in a control subject.

In the context of the invention, liver-specific insulin resistance or hepatic insulin resistance may be defined as the inability of insulin to suppress hepatic glucose concentration and could be assessed using a hyperinsulinemic clamp while injecting a stable-isotope or radioactively labeled glucose. Hyperinsulinemic clamp is later on defined herein.. The opposite of liver-specific insulin resistance is hepatic insulin sensitivity, which is defined as a decrease in endogenous glucose production during hyperinsulinemia.

The use of a MBL2 as a biomarker of liver-specific insulin resistance is more attractive than the use of other known methods such as hyperinsulinemic clamp while injecting stable-isotope or radioactively labeled glucose. Technology for analyzing stable-isotope labeled glucose is often not available and use of radiolabeled glucose is prohibited by ethical consideration. It would make assessment of hepatic insulin resistance more accessible to a wider range of research and medical settings.

In the context of the invention, diagnosis preferably means a predictive risk assessment of the subsequent development of liver-specific insulin resistance in a subj ect.

In the context of the invention, a subject may be an animal or a human being. In principle, any subject could be diagnosed using the method of the invention. The diagnosis method may be applied as often as necessary in a subject. Preferably, a subject diagnosed is a subject suspected to have a high risk of developing a liver-specific insulin resistance, due for example to potential genetic predisposition, and/or to the age of the subject and/or to the lifestyle of a subject (for example nutritional habit and/or to the absence of physical activity). Preferably, a subject is a human being.

In the context of the invention, "a MBL2 gene or MBL2 nucleotide molecule or MBL2 nucleic acid as identified herein" preferably means a gene or nucleotide molecule represented by a nucleotide sequence having at least 60% identity with SEQ ID NO:1.

In the context of the invention, "a polypeptide or protein as identified herein" preferably means a polypeptide encoded by a gene or nucleotide molecule as identified herein.

In the context of the invention, "a reference value" for the expression level of a MBL2 gene as identified herein is preferably the average value for said expression level in a control subject. More preferably, a control subject is a subject, who has not developed a liver-specific insulin resistance as diagnosed by any of the methods as mentioned earlier herein. Alternatively according to an even more preferred embodiment, a control subject is a subject who has not developed any of the characteristics (i.e. parameters) of a liver-specific insulin resistance yet. For example, a subject will not be said to have any symptom associated with hyperinsulinemia.

The assessment of the expression level of a gene as identified herein may be realised at the protein expression level (quantifying the amount of a protein encoded by said genes as identified herein) and/or by quantifying the amount of a gene (or nucleotide molecule) encoding said protein (both the reference value from a control subject and the value from a subject wherein the method is being carried out). Insulin is able to down-regulate the expression of plasma MBL2 in healthy subjects whereas insulin does not affect the expression of plasma MBL2 in patients with a type 2 diabetic patient. The expression of MBL2 is specifically expressed in human hepatic tissue, is secreted and detectable in blood, preferably plasma. Each of these features renders this gene attractive to be used as a marker for liver-specific insulin resistance. The invention encompasses the use of a MBL2 gene represented by a sequence having at least 60% identity with SEQ ID NO:1.

The skilled person will understand that for the MBL2 gene (or nucleotide sequence) and corresponding polypeptide or protein, it is possible to isolate multiple isoforms of a given protein depending on the subject to be tested. It is to be understood that each gene as identified herein by a given Sequence Identity Number (SEQ ID NO) is not limited to this specific sequence. Throughout this application, each time one refers to a specific nucleotide sequence SEQ ID NO (take SEQ ID NO:1 as example), one may replace it by:
i. a polypeptide comprising an amino acid sequence that has at least 60% sequence identity with amino acid sequence SEQ ID N0:2 as being encoded by SEQ ID NO:1,
ii.a nucleotide sequence comprising a nucleotide sequence that has at least 60% sequence identity with SEQ ID NO: 1;
iii. a nucleotide sequence the complementary strand of which hybridizes to a nucleotide sequence of (ii);
iv. a nucleotide sequence the sequence of which differs from the sequence of a nucleotide sequence of (iii) due to the degeneracy of the genetic code.
iv. a nucleotide sequence that encodes an amino acid sequence that has at least 60% amino acid identity with an amino acid sequence encoded by a nucleotide sequence SEQ ID NO:1.

Each nucleotide sequence or amino acid sequence described herein by virtue of its identity percentage (at least 60%) with a given nucleotide sequence or amino acid sequence respectively has in a further preferred embodiment an identity of at least 65%, 70%, 75%, 80%, 85%, 90%, 95%, 97%, 98%, 99% or more identity with the given nucleotide or amino acid sequence respectively. In a preferred embodiment, sequence identity is determined by comparing the whole length of the sequences as identified herein. Identity is later herein defined.

Each of the quantification methods as identified in this paragraph is preferably for use for assessing a MBL2 activity. The quantification of the amount of a gene (or nucleotide molecule) as identified herein is preferably performed using classical molecular biology techniques such as (real time) PCR, arrays or northern analysis. In this embodiment, a gene (or nucleotide molecule) encoding a polypeptide as defined herein means a messenger RNA (mRNA). Alternatively, according to another preferred embodiment, in a diagnosis method the expression level of a polypeptide is determined directly by quantifying the amount of said polypeptide. Quantifying a polypeptide amount may be carried out by any known technique. Preferably, a polypeptide amount is quantified using a molecule which specifically binds to said polypeptide. Preferred binding molecules are selected from: an antibody, which has been specifically raised for recognizing a given polypeptide as preferably identified in the experimental part, any other molecule which is known to specifically bind said polypeptide. Such antibody could be used in any immunoassay known to the skilled person such as western blotting, or ELISA (Enzyme-Linked Immuno Sorbent Assay) or FACS (Fluorescence Activated Cell Sorting) using latex beads. In a preferred embodiment, a MBL2 polypeptide is quantified using an MBL2 oligomer ELISA kit as described in the experimental data. The preparation of an antibody is known to those skilled in the art. A short explanation of methods that could be used to prepare antibodies is later herein given. In the context of the invention, any other molecule known to bind a given polypeptide may be a nucleic acid, e.g. a DNA regulatory region, a polypeptide, a metabolite, a substrate, a regulatory element, a structural component, a chaperone (transport) molecule, a peptide mimetic, a non-peptide mimetic, or any other type of ligand. Mimetic is later herein defined. Binding of a given polypeptide to a second binding molecule may be detected by any standard methods known to those skilled in the art. Suitable methods include affinity chromatography co-electrophoresis (ACE) assays and ELISA. The skilled person will understand that alternatively or in combination with the quantification of a gene encoding a given polypeptide and/or the corresponding polypeptide, the quantification of a substrate of the corresponding polypeptide or of any compound known to be associated with the function of the corresponding polypeptide or the quantification of the function or activity of the corresponding polypeptide using a specific assay is encompassed within the scope of the diagnosis method of the invention. For example, transactivation of a target gene by a MBL2 binding molecule can be determined and quantified, e.g., in a transient transfection assay in which the promoter of the target gene is linked to a reporter gene, e.g., P-galactosidase or luciferase. Such evaluations can be done *in vitro* or *in vivo* or *ex vivo.*

Since the expression level of a gene (or nucleotide molecule) encoding a polypeptide as identified herein and/or amounts of corresponding polypeptide may be difficult to detect in a subject, a sample from a subject is preferably used. According to another preferred embodiment, the expression level (of a gene or nucleotide molecule or polypeptide) is determined *ex vivo* in a sample obtained from a subject. A sample preferably comprises or consists of a solid of a semi solid sample. Alternatively, a sample preferably comprises or consists of a fluid obtained from a subject. More preferably, a fluid comprises or consists of or is selected from: urine, faeces, blood or saliva. Even more preferably, a fluid is blood. Most preferably, a fluid comprises or consists of plasma. Subsequently, a nucleotide molecule encoding a polypeptide as identified herein and/or said polypeptide are extracted and optionally purified using known methods to the skilled person.

In a preferred embodiment, the quantification or the presence of a MBL2 polypeptide is assessed in the plasma of a single subject before and after hyperinsulinemic clamp. Assessing the presence of a MBL2 in the plasma of a single subject before and after hyperinsulinemic clamp could be replaced by assessing the presence of a MBL2 in the plasma of a single subject during hyperinsulinemic clamp. This method is preferably applied for diagnosing a liver-specific insulin resistance. This is a method known to the skilled person. This method could be applied as explained in the experimental part. A subject is preferably said to be diagnosed as having a liver-specific insulin resistance when the concentration of plasma MBL2 has not been decreased before and after an hyperinsulinemic clamp. In a more preferred embodiment, a subject is preferably said to be diagnosed as having a liver-specific insulin resistance when the concentration of plasma MBL2 has not been significantly decreased before and after an hyperinsulinemic clamp using a statistic test. A preferred statistic test used in this context is defined in the experimental part.

In a more preferred diagnosis method, a liver-specific insulin resistance is diagnosed when the comparison leads to the finding of a detectable expression of a MBL2 gene (or nucleotide molecule) and/or of a corresponding polypeptide as identified herein. Alternatively or in combination with earlier preferred embodiment, the comparison leads to the finding of an increase of the expression level of a MBL2 gene (or nucleotide molecule) and/or of a corresponding polypeptide as identified herein. In control subjects as defined before, the change in the expression of said gene (or nucleotide molecule) and/or corresponding polypeptide during a hyperinsulinemic clampis preferably significantly lower than in subjects diagnosed as having a liver-specific insulin resistance. Detection or an increase of the expression level of a polypeptide as identified herein and/or an increase or a detection of the expression level of a gene (or nucleotide molecule) encoding said polypeptide (or steady state level of said polypeptide) is preferably defined as being a detectable change of the expression level of said polypeptide and/or of a nucleotide molecule encoding said polypeptide (or steady state level of the encoded polypeptide or any detectable change in the biological activity of a polypeptide as defined herein) using a method as defined earlier on as compared to the expression level of a polypeptide as identified herein and/or of a corresponding gene (or nucleotide molecule) (or steady state level of the corresponding encoded polypeptide) in a control subject. According to a preferred embodiment, detection or an increase of the expression level of a gene (or nucleotide molecule) as identified herein is quantified using a specific mRNA assay for the gene (or nucleotide molecule) as earlier defined herein. Preferably, an increase of the expression level of a gene (or nucleotide molecule) encoding a polypeptide as identified herein means an increase of at least 5% of the expression level of the gene (or nucleotide molecule) using PCR. For example, preferred primers used for the PCR for the detection of the expression of a MBL2 gene are identified as
SEQ ID NO:3:
   GCAAACAGAAATGGCACGTATC
and SEQ ID NO:4.:
   CTGGAACTTGACACACAAGGC
ore preferably, an increase of the expression level of a gene (or nucleotide molecule) means an increase of at least 10%, even more preferably at least 20%, at least 30%, at least 40%, at least 50%, at least 70%, at least 90%, at least 150% or more.

Preferably, an increase of the expression level of a polypeptide as identified herein means an increase of at least 5% of the expression level of said polypeptide using western blotting and/or using ELISA or a suitable assay. More preferably, an increase of the expression level of a polypeptide means an increase of at least 10%, even more preferably at least 20%, at least 30%, at least 40%, at least 50%, at least 70%, at least 90%, at least 150% or more.

Preferably, an increase of an activity of a given polypeptide as identified herein means an increase of at least 5% of the polypeptide activity using a suitable assay. More preferably, an increase of a polypeptide activity means an increase of at least 10%, even more preferably at least 20%, at least 30%, at least 40%, at least 50%, at least 70%, at least 90%, at least 150% or more.

In a preferred aspect of the invention, a liver-specific insulin resistance is diagnosed using an hyperinsulinemic clamp as earlier defined since this is the most specific way of assessing whether a given MBL2 concentration is indicative of said disease or condition.

In another preferred aspect of the invention, MBL2 is used as a single biomarker indicative of a hepatic PPAR-α activity. This is quite an attractive method to rely on one single biomarker to give a reliable estimation of the diagnosis of a disease or condition as identified herein. Moreover, this biomarker is easy to detect in plasma and is only expressed in the liver.

### Method for identification

In a further aspect, there is provided a method for identification of a substance capable of preventing, treating and/or delaying the progression of liver-specific insulin resistance in a subject, the method comprising the steps of:
(a) providing a test cell population or a test animal capable of expressing a gene (or nucleotide molecule) encoding a polypeptide as identified herein and/or a gene (or nucleotide molecule) encoding said polypeptide;
(b) contacting the test cell population or the test animal with a substance;
(c) determining the expression level of a gene (or nucleotide molecule) encoding said polypeptide or the activity or steady state level of said polypeptide in a test cell population or in the test animal contacted with the substance;
(d) comparing the expression, activity or steady state level determined in (c) with the expression, activity or steady state level of the gene (or nucleotide molecule) or of the polypeptide in a test cell population or in a test animal that is not contacted with the substance; and,
(e) identifying a substance that produces a difference in expression level, activity or steady state level of the gene (or nucleotide molecule) or the polypeptide, between the test cell population or test animal that is contacted with the substance and the test cell population or test animal that is not contacted with the substance.

In a preferred embodiment, a test animal is a mouse. A preferred test cell population comprises mammalian cells, more preferably human cells. Even more preferred cells are hepatic cells, human hepatic cells such as hepatocytes. Alternatively, a human hepatoma cell line such as HepG2 could be used.

Alternatively or in combination with previous preferred embodiment in a further preferred embodiment, in step (a), a test cell or a test animal has been modified to overexpress a polypeptide as identified herein. This is preferably carried out by transforming a test cell with a nucleic acid construct comprising a nucleotide sequence encoding said polypeptide as defined herein. Alternatively, this is preferably carried out by generating a test animal being transgenic for a given polypeptide as identified herein and as later explained herein. In a further aspect, the invention relates to such a nucleic acid construct. Preferably, a nucleotide sequence is operably linked to a promoter that is capable of driving expression of a nucleotide sequence in a chosen test cell. In a preferred embodiment a nucleic acid construct is a viral gene therapy vector selected from gene therapy vectors based on an adenovirus, an adeno-associated virus (AAV), a herpes virus, a pox virus and a retrovirus. A preferred viral gene therapy vector is an AAV or Lentiviral vector. Such vectors are further described herein below.

Depending on the system used (test cell or test animal), the skilled person will know which conditions are preferred for the contacting step (b).

In step (c), the expression level of a gene (or nucleotide molecule) encoding a polypeptide as identified herein or the activity or steady state level of said polypeptide may be carried out as earlier herein defined.

In a preferred method in step (e), the difference identified in step (d) produced by the substance is a decrease of the expression level of said corresponding gene (or nucleotide molecule), or of the activity or steady state level of said polypeptide.

A decrease of the expression level of a gene (or nucleotide molecule) encoding a polypeptide as identified herein (or steady state level of said polypeptide) is preferably defined as being a detectable change of the expression level of said polypeptide and/or of a gene (or nucleotide molecule) encoding said polypeptide (or steady state level of the encoded polypeptide) or any detectable change in a biological activity of said polypeptide using a method as defined earlier on as compared to the expression level of a given polypeptide and/or of a corresponding gene (or nucleotide molecule) (or steady state level of the corresponding encoded polypeptide in a control subject. According to a preferred embodiment, a decrease of the expression level of a gene (or nucleotide molecule) encoding a given polypeptide as identified herein is quantified using a specific mRNA assay for corresponding gene as earlier defined herein. Preferably, a decrease of the expression level of a gene (or nucleotide molecule) encoding a given polypeptide means a decrease of at least 5% of the expression level of the gene (or nucleotide molecule) using PCR. Preferred primers used for the PCR are already identified herein. More preferably, a decrease of the expression level of a gene (or nucleotide molecule) means a decrease of at least 10%, even more preferably at least 20%, at least 30%, at least 40%, at least 50%, at least 70%, at least 90%, at least 150% or more.

Preferably, a decrease of the expression level of a given polypeptide means a decrease of at least 5% of the expression level of said polypeptide using western blotting and/or using ELISA or a suitable assay. More preferably, a decrease of the expression level of a polypeptide means a decrease of at least 10%, even more preferably at least 20%, at least 30%, at least 40%, at least 50%, at least 70%, at least 90%, at least 150% or more.

Preferably, a decrease of an activity of a given polypeptide means a decrease of at least 5% of the polypeptide activity using a suitable assay. More preferably, a decrease of the polypeptide activity means a decrease of at least 10%, even more preferably at least 20%, at least 30%, at least 40%, at least 50%, at least 70%, at least 90%, at least 150% or more.

In a most preferred method for identifying a substance capable of preventing, treating and/or delaying the progression of liver-specific insulin resistance in a subject when the comparison leads to the comparison leads to a decrease of the expression level of a gene (or nucleotide molecule) as identified herein, said decrease being detected at the level of a gene (or nucleotide molecule) (a decrease of at least 5% of the expression level of the gene (or nucleotide molecule)) using PCR primers as defined herein.

Preferred genes and corresponding polypeptides have already been defined herein.

In one further aspect, the invention also pertains to a substance that is identified in a aforementioned method.

### Sequence identity

"Sequence identity" is herein defined as a relationship between two or more amino acid (polypeptide or protein) sequences or two or more nucleic acid (polynucleotide) sequences, as determined by comparing the sequences. The whole SEQ ID NO may be used or part thereof. In a preferred embodiment, the whole SEQ ID NO as identified herein is used. In the art, "identity" also means the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods, including but not limited to those described in (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48:1073 (1988).

Preferred methods to determine identity are designed to give the largest match between the sequences tested. Methods to determine identity and similarity are codified in publicly available computer programs. Preferred computer program methods to determine identity and similarity between two sequences include e.g. the GCG program package (Devereux, J., et al., Nucleic Acids Research 12 (1): 387 (1984)), BestFit, BLASTP, BLASTN, and FASTA (Altschul, S. F. et al., J. Mol. Biol. 215:403-410 (1990). The BLAST X program is publicly available from NCBI and other sources (BLAST Manual, Altschul, S., et al., NCBI NLM NIH Bethesda, MD 20894; Altschul, S., et al., J. Mol. Biol. 215:403-410 (1990). The well-known Smith Waterman algorithm may also be used to determine identity.

Preferred parameters for polypeptide sequence comparison include the following: Algorithm: Needleman and Wunsch, J. Mol. Biol. 48:443-453 (1970); Comparison matrix: BLOSSUM62 from Hentikoff and Hentikoff, Proc. Natl. Acad. Sci. USA. 89:10915-10919 (1992); Gap Penalty: 12; and Gap Length Penalty: 4. A program useful with these parameters is publicly available as the "Ogap" program from Genetics Computer Group, located in Madison, WI. The aforementioned parameters are the default parameters for amino acid comparisons (along with no penalty for end gaps).

Preferred parameters for nucleic acid comparison include the following: Algorithm: Needleman and Wunsch, J. Mol. Biol. 48:443-453 (1970); Comparison matrix: matches=+10, mismatch=0; Gap Penalty: 50; Gap Length Penalty: 3. Available as the Gap program from Genetics Computer Group, located in Madison, Wis. Given above are the default parameters for nucleic acid comparisons.

Optionally, in determining the degree of amino acid similarity, the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to Ser; Arg to Lys; Asn to G1n or His; Asp to Glu; Cys to Ser or Ala; G1n to Asn; Glu to Asp; Gly to Pro; His to Asn or G1n; Ile to Leu or Val; Leu to Ile or Val; Lys to Arg, G1n or Glu; Met to Leu or Ile; Phe to Met, Leu or Tyr; Ser to Thr; Thr to Ser; Trp to Tyr; Tyr to Trp or Phe; and Val to Ile or Leu.

### Antibodies

Some aspects of the invention concern the use of an antibody or antibody-fragment that specifically binds to a polypeptide as identified herein. Methods for generating antibodies or antibody-fragments that specifically bind to a polypeptide are described in e.g. Harlow and Lane (1988, Antibodies: A Laboratory Manual, Cold Spring Harbor Laboratory Press, Cold Spring Harbor, NY) and WO 91/19818; WO 91/18989; WO 92/01047; WO 92/06204; WO 92/18619; and US 6,420,113 and references cited therein. The term "specific binding," as used herein, includes both low and high affinity specific binding. Specific binding can be exhibited, e.g., by a low affinity antibody or antibody-fragment having a Kd of at least about 10⁻⁴ M. Specific binding also can be exhibited by a high affinity antibody or antibody-fragment, for example, an antibody or antibody-fragment having a Kd of at least about of 10-⁷ M, at least about 10⁻⁸ M, at least about 10⁻⁹ M, at least about 10⁻¹⁰ M, or can have a Kd of at least about 10⁻¹¹ M or 10⁻¹² M or greater.

### Recombinant techniques and methods for over-expression of a polypeptide as identified herein in a test cell or in a test animal

A polypeptide for use in the present invention can be prepared using recombinant techniques, in which a gene (or nucleotide molecule) encoding said polypeptide of interest is (over)expressed in a suitable host cell. The present invention thus also concerns the use of a vector comprising a nucleic acid molecule as defined above. Preferably the vector is a replicative vector comprising on origin of replication (or autonomously replication sequence) that ensures multiplication of the vector in a suitable host for the vector. Alternatively a vector is capable of integrating into a host cell's genome, e.g. through homologous recombination or otherwise. A particularly preferred vector is an expression vector wherein a nucleotide molecule encoding a polypeptide as defined above, is operably linked to a promoter capable of directing expression of the coding sequence in a host cell for the vector.

As used herein, the term "promoter" refers to a nucleic acid fragment that functions to control the transcription of one or more genes, located upstream with respect to the direction of transcription of the transcription initiation site of the gene, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active under most physiological and developmental conditions. An "inducible" promoter is a promoter that is regulated depending on physiological or developmental conditions. A "tissue specific" promoter is only active in specific types of differentiated cells/tissues, such as preferably a monocyte or a macrophage cell or tissue derived there from.

Expression vectors allow a polypeptide of the invention as defined above to be prepared using recombinant techniques in which a nucleotide molecule encoding said polypeptide of interest is expressed in a suitable cell, e.g. cultured cells or cells of a multicellular organism, such as described in Ausubel et al., "Current Protocols in Molecular Biology", Greene Publishing and Wiley-Interscience, New York (1987) and in Sambrook and Russell (2001, *supra);* both of which are incorporated herein by reference in their entirety. Also see, Kunkel (1985) Proc. Natl. Acad. Sci. 82:488 (describing site directed mutagenesis) and Roberts et al. (1987) Nature 328:731-734 or Wells, J.A., et al. (1985) Gene 34: 315 (describing cassette mutagenesis).

Typically, a nucleotide molecule encoding a desired polypeptide is used in an expression vector. The phrase "expression vector" generally refers to a nucleotide molecule represented by a nucleotide sequence that is capable of effecting expression of a gene in hosts compatible with such sequences. These expression vectors typically include at least suitable promoter sequences and optionally, transcription termination signals. Additional factors necessary or helpful in effecting expression can also be used as described herein. A nucleic acid or DNA encoding a polypeptide is incorporated into a DNA construct capable of introduction into and expression in an *in vitro* cell culture. Specifically, DNA constructs are suitable for replication in a prokaryotic host, such as bacteria, *e.g., E. coli,* or can be introduced into a cultured mammalian, plant, insect, e.g., Sf9, yeast, fungi or other eukaryotic cell lines.

DNA constructs prepared for introduction into a particular host typically include a replication system recognized by the host, the intended DNA segment encoding the desired polypeptide, and transcriptional and translational initiation and termination regulatory sequences operably linked to the polypeptide-encoding segment. A DNA segment is "operably linked" when it is placed into a functional relationship with another DNA segment. For example, a promoter or enhancer is operably linked to a coding sequence if it stimulates the transcription of the sequence. DNA for a signal sequence is operably linked to DNA encoding a polypeptide if it is expressed as a pre-protein that participates in the secretion of the polypeptide. Generally, DNA sequences that are operably linked are contiguous, and, in the case of a signal sequence, both contiguous and in reading phase. However, enhancers need not be contiguous with the coding sequences whose transcription they control. Linking is accomplished by ligation at convenient restriction sites or at adapters or linkers inserted in lieu thereof.

The selection of an appropriate promoter sequence generally depends upon the host cell selected for the expression of the DNA segment. Examples of suitable promoter sequences include prokaryotic, and eukaryotic promoters well known in the art (see, e.g. Sambrook and Russell, 2001, *supra).* The transcriptional regulatory sequences typically include a heterologous enhancer or promoter that is recognised by the host. The selection of an appropriate promoter depends upon the host, but promoters such as the trp, lac and phage promoters, tRNA promoters and glycolytic enzyme promoters are known and available (see, e.g. Sambrook and Russell, 2001, *supra).* Expression vectors include the replication system and transcriptional and translational regulatory sequences together with the insertion site for the polypeptide encoding segment can be employed. Examples of workable combinations of cell lines and expression vectors are described in Sambrook and Russell (2001, *supra)* and in Metzger et al. (1988) Nature 334: 31-36. For example, suitable expression vectors can be expressed in, yeast, *e.g. S. cerevisiae, e.g.,* insect cells, *e.g.,* Sf9 cells, mammalian cells, *e.g.,* CHO cells and bacterial cells, *e.g., E. coli.* The host cells may thus be prokaryotic or eukarotic host cells. A host cell may be a host cell that is suitable for culture in liquid or on solid media. A host cell is preferably used in a method for producing a polypeptide of the invention as defined above or in a method for identification of a substance as defined herein. Said method comprises the step of culturing a host cell under conditions conducive to the expression of a polypeptide. Optionally the method may comprise recovery of a polypeptide. A polypeptide may e.g. be recovered from the culture medium by standard protein purification techniques, including a variety of chromatography methods known in the art per se.

Alternatively, a host cell is a cell that is part of a multi-cellular organism such as a transgenic plant or animal, preferably a non-human animal. A transgenic plant comprises in at least a part of its cells a vector as defined above. Methods for generating transgenic plants are e.g. described in U.S. 6,359,196 and in the references cited therein. Such transgenic plant or animal may be used in a method for producing a polypeptide of the invention as defined above and/or in a method for identification of a substance both as defined herein. For transgenic plant, the method comprises the step of recovering a part of a transgenic plant comprising in its cells the vector or a part of a descendant of such transgenic plant, whereby the plant part contains said polypeptide, and, optionally recovery of said polypeptide from the plant part. Such methods are also described in U.S. 6,359,196 and in the references cited therein. Similarly, the transgenic animal comprises in its somatic and germ cells a vector as defined above. The transgenic animal preferably is a non-human animal. More preferably, a non-human animal is a mouse. Methods for generating transgenic animals are e.g. described in WO 01/57079 and in the references cited therein. Such transgenic animals may be used in a method for producing a polypeptide as defined herein, said method comprising the step of recovering a body fluid from a transgenic animal comprising the vector or a female descendant thereof, wherein the body fluid contains said polypeptide, and, optionally recovery of said polypeptide from the body fluid. Such methods are also described in WO 01/57079 and in the references cited therein. The body fluid containing said polypeptide preferably is blood or more preferably milk.

Another method for preparing a polypeptide is to employ *an in nitro* transcription/translation system. DNA encoding a polypeptide is cloned into an expression vector as described *supra.* The expression vector is then transcribed and translated *in vitro.* The translation product can be used directly or first purified. A polypeptide resulting from *in vitro* translation typically does not contain the post-translation modifications present on polypeptides synthesised *in vivo,* although due to the inherent presence of microsomes some post-translational modification may occur. Methods for synthesis of polypeptides by *in vitro* translation are described by, for example, Berger & Kimmel, Methods in Enzymology, Volume 152, Guide to Molecular Cloning Techniques, Academic Press, Inc., San Diego, CA, 1987.

In this document and in its claims, the verb "to comprise" and its conjugations is used in its non-limiting sense to mean that items following the word are included, but items not specifically mentioned are not excluded. In addition the verb "to consist" may be replaced by "to consist essentially of' meaning that a polypeptide or a nucleic acid construct or an antibody as defined herein may comprise additional component(s) than the ones specifically identified, said additional component(s) not altering the unique characteristic of the invention. In addition, reference to an element by the indefinite article "a" or "an" does not exclude the possibility that more than one of the element is present, unless the context clearly requires that there be one and only one of the elements. The indefinite article "a" or "an" thus usually means "at least one".
Each embodiment as identified herein may be combined together unless otherwise indicated. All patent and literature references cited in the present specification are hereby incorporated by reference in their entirety.

The invention is further illustrated by the following examples which should not be construed for limiting the scope of the present invention.

### Description of the figures

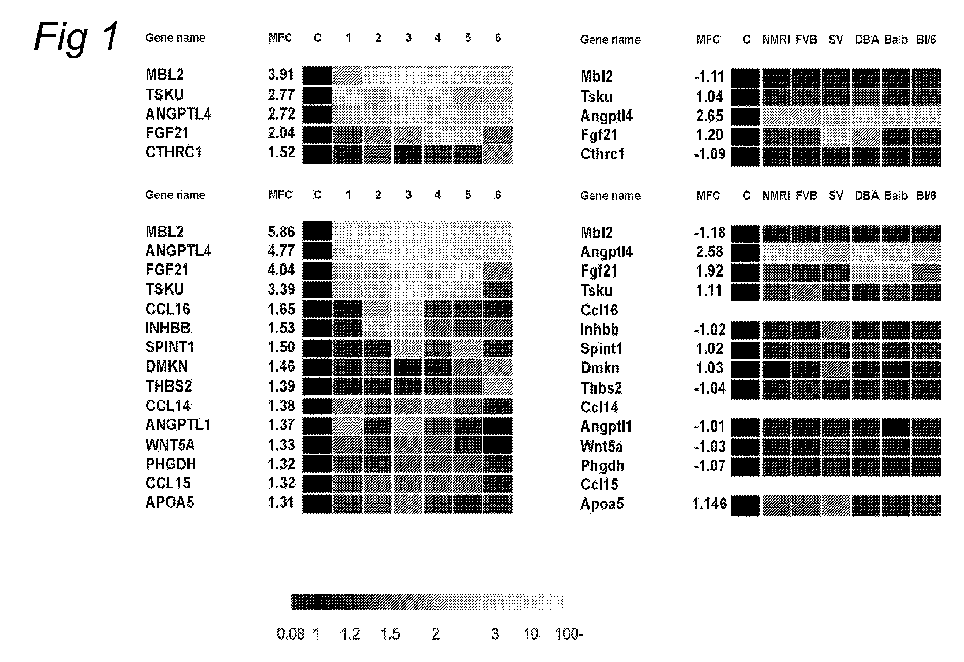

**Figure 1****. Gene encoding secreted proteins regulated by PPARα in human liver.** Heat map illustrating the relative induction of genes encoding secreted proteins in response to 6h (top panel) or 24h (bottom panel) Wy14643 treatment in human hepatocytes. Relative changes in expression of the corresponding mouse orthologs in mouse hepatocytes are shown in parallel. All genes were significantly changed (P < 0.05) and were ranked based on mean fold-change (MFC). Expression levels in the DMSO-treated cells were set at 1. Genes encoding secreted proteins were selected using Gene Ontology Classification, SignalP and ngLOC (*n*-gram-based Bayesian classifier) predicting tools.

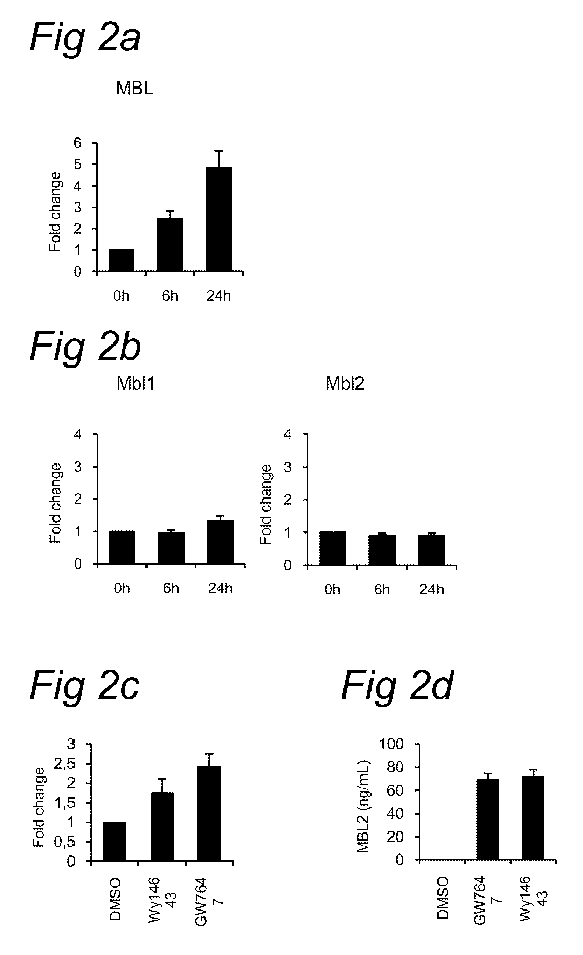

**Figure 2****. PPARα regulates MBL2 expression in human liver.** (A) Relative induction of Mannose binding lectin (MBL2) by Wy14643 (50 µM) in human primary hepatocytes. (B) Relative induction of Mannose binding lectin 1 (Mbl1) and Mannose binding lectin 2 (Mb12) by Wy14643 (10 µM) in mouse primary hepatocytes. (C) Relative induction of MBL2 by Wy14643 (10 µM) and GW7647 (10 µM) for 6h in HepG2 cell line. Expression of cells treated with DMSO was set at 1. Gene expression was determined by qPCR. Error bars represent SEM.
   (D) MBL2 protein concentration in the serum free medium of HepG2 cells incubated 24h in the presence or absence of Wy14643 and GW7647 as assessed by MBL oligomer ELISA kit using biotinylated monoclonal detection antibody. Error bars represent SEM. ND (Not detected).

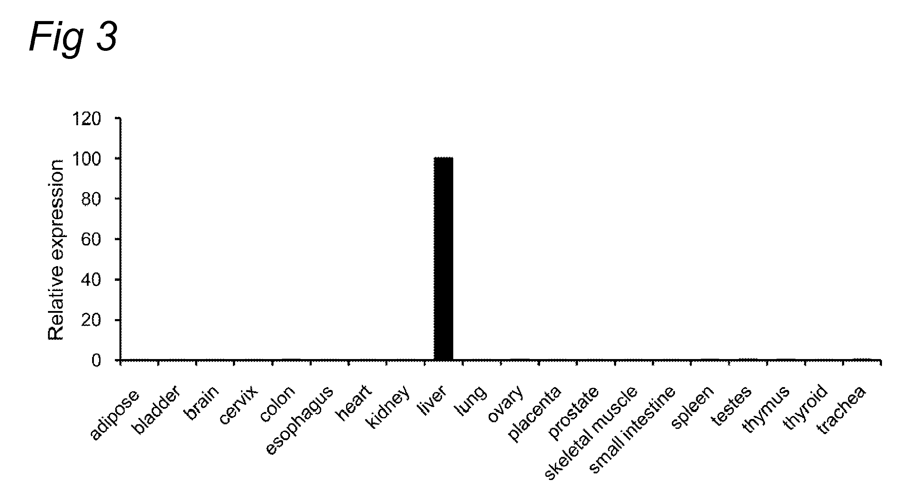

**Figure 3****. MBL2 is expressed specifically in human liver.** mRNA expression of MBL2 was determined in human tissues by Q-PCR. Human RNA represented a mix from several individuals (AMBION, First choice human total RNA). Expression levels were related to the liver which was the tissue showing highest expression.

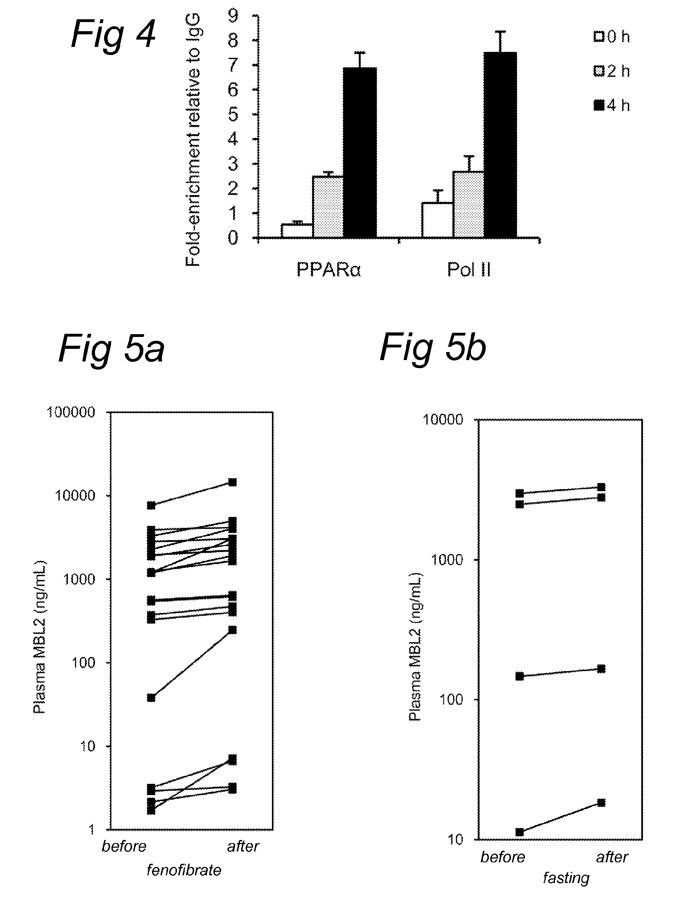

**Figure 4****. PPARα and RNA polymerase II (POL II) bind directly to the MBL2 transcription starting site (TSS).** Chromatin immunoprecipitation of MBL2 transcription starting site (TSS) using antibodies against PPARα and POL II in HepG2 cells treated with Wy14643 (50µM) for 2 and 4h.
**Figure 5****. Plasma levels of MBL2 are increased by fenofibrate treatment and fasting.** (A) Quantification of the effect of 6 weeks fenofibrate (200 mg/day micronized; Lipanthyl) treatment on plasma MBL2 concentrations in 19 subjects with the metabolic syndrome. (B) Quantification of the effect of 48h of fasting on plasma MBL2 concentrations in 3 healthy males. Quantification assessed by using MBL Oligomer ELISA kit.

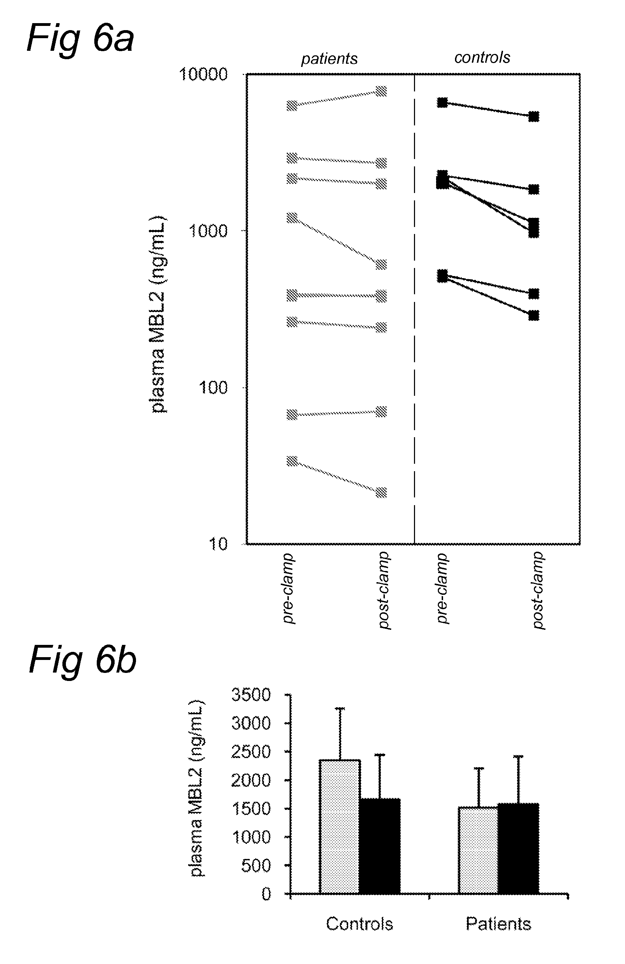

Figure 6. Insulin suppresses MBL2 plasma concentration. Changes of MBL2 concentrations before and after 180 minutes of isoglycemic-hyperinsulinemic clamp in a group of 6 healthy controls and nine T2DM patients (A). Mean concentration of plasma MB2 levels of the controls and patients before and after 180 minutes of isoglycemic-hyperinsulinemic clamp (B). Error bars represent SEM. *P < 0.01 according to Student's T-test. Quantification assessed by using MBL Oligomer ELISA kit.

### Examples

### Results:

### MBL2 expression in liver is regulated by PPARα in human specific manner

The aim of the present paper was to screen for novel circulating mediators of PPARα activity in human. To that end, we treated primary human hepatocytes with the PPARα agonist Wy14643 for 6 hours and performed Affymetrix microarray analysis. Differentially expressed genes encoding secreted proteins were selected using Gene Ontology Classification. In addition to established secreted targets of PPARα such as ANGPTL4 and FGF21, expression of MBL2 was significantly upregulated by PPARα activation in all donors (Figure 1). In fact, MBL2 represented the most highly induced gene encoding a secreted protein after both 6 hour and 24 hour. Wy14743 incubation. Induction of MBL2 was confirmed by PCR and was further augmented after 24 hours of PPARα activation (Figure 2A). In contrast to human hepatocytes, incubation of mouse hepatocytes with Wy14643 did not result in significant induction of MBL2 (Figure 2B). It should be emphasized that mouse expresses two MBL2 isomers: Mbl1 and Mbl2.
Induction of MBL2 by PPARα activation could be reproduced in HepG2 cells. Treatment of the cells with Wy14643 and GW7647 significantly increased MBL2 gene expression levels (Figure 2C) as well secretion into the medium (Figure 2D). To investigate whether MBL2 expression may be regulated by PPARs in other tissues, we first screened a panel of organs for the presence of MBL2 mRNA. MBL2 expression was exclusive to liver among a panel of 20 human tissues (Figure 3). Overall, these data demonstrate that MBL2 expression and secretion are induced by PPARα in human liver.

### MBL2 is a direct target gene of human PPARα

Considering its robust induction by PPARα agonist in primary human hepatocytes and HepG2 cells, MBL2 likely represents a direct PPARα target gene. Direct PPAR target genes identified thus far are characterized by a PPAR response elements (PPRE) in their promoter region. To locate a putative PPREs we screened 5 kb up and downstream of the transcriptional start site of MBL2 using an in silico screening method [9]. However, no putative PPREs could be identified (data not shown). Recent studies suggest that many PPAR binding sites are located outside the upstream promoter area and may contact the general transcriptional machinery and transcription start site (TSS) via DNA looping [10, 11]. Therefore to determine whether MBL2 may represent a target gene of PPARα, chromatin immuneprecipitation (ChIP) was performed on the TSS using antibodies against PPARα and Pol II. In human HepG2 cells, binding of PPARα to the transcription starting site was markedly increased after 2h of Wy14643 treatment and further enhanced after 4h of Wy14643 (Figure 4). Binding of RNA pol II was also increased by Wy14643 and mirrored the profile for PPARα. Overall, our data suggest that MBL2 is a direct PPARα target gene in human liver.

### Human MBL2 plasma levels are increased by fenofibrate

Since our aim was to find novel circulating mediators of PPARα activity in human, it was important to show that circulating MBL2 levels are increased by PPARα activation. Accordingly, we measured plasma levels of MBL2 protein in 19 subjects with the metabolic syndrome before and after 6 weeks of treatment with the PPARα agonist fenofibrate (200 mg/day micronized; Lipanthyl). As previously shown, we found a very large inter-individual variation in fasting plasma MBL2 levels. In spite of the large variation in baseline MBL2 levels, fenofibrate raised plasma MBL2 in all subjects (Figure 5A). The mean increase in plasma MBL2 was calculated at 86% (P<0.01). The data show that circulating MBL2 levels are increased by PPARα activation in human.
In mice and possibly in human, hepatic PPARα can be activated by fasting [12]. Accordingly, we studied the effect of a 48 fast on plasma MBL2 levels in 3 subjects. Although the number of subjects was clearly limited, MBL2 consistently went up upon fasting in all subjects (Figure 5B) (P<0.05).

### Insulin lowers plasma MBL2 levels

To further understand the regulation of plasma MBL2 levels and its relation to metabolism, we measured plasma MBL2 in a group of healthy subjects undergoing a hyperinsulinemic clamp. During the clamp plasma MBL2 levels decreased in healthy control subjects (29.1 %, P<0.01). In contrast, plasma MBL2 levels did not significantly change in type T2DM patients (Figure 6). These data suggests that insulin suppresses plasma MBL2 in insulin-sensitive but not insulin resistant subjects. Additionally, the data suggest that changes in plasma MBL2 may have potential as biomarker of insulin sensitivity.

### Discussion

MBL2 is an important player in complement cascade activation as part of the first line host defense. In the present paper we show that: 1) MBL2 is a direct target gene of PPARα in human hepatocytes and 2) plasma MBL2 levels are increased by chronic PPARα activation via fibrate drugs. MBL2 may thus represent a novel circulating mediator of PPARα action.
MBL2 recognizes and binds to conserved carbohydrate structures present on the surface of microorganisms. MBL2 binding results in activation of the lectin pathway of the complement system by the action of MBL2-associated serine proteases (MASPs), which associate with circulating MBL2 in their inactive proenzymatic forms [13]. Alternatively, MBL2 acts as an opsonin, leading to stimulation of phagocytosis by binding to cell-surface receptors present on phagocytic cells. A wealth of data published in the past decade show that in addition to being a crucial regulator of hepatic lipid metabolism, PPARα also has a major impact on inflammatory pathways [14]. The pronounced induction of MBL2 by PPARα in human liver fits within the role of PPARα as important regulator of inflammation and innate immunity.
Currently, little is known about factors controlling plasma MBL2 levels. While MBL2 levels in serum are known to be largely determined by polymorphisms in the MBL2 gene, differences in plasma MBL2 of up to 10-fold can be found between individuals despite identical genotypes [15]. Also, little is known about regulation of MBL2 gene expression. The specific expression of MBL2 in liver has been suggested to be mediated by HNF3 based on the presence of specific response element in the MBL2 promoter and its ability to bind HNF3 in vitro [16]. However, extensive evidence for regulation of MBL2 by HNF3 is currently lacking. Clearly, regulation of MBL2 by PPARα does not exclude regulation by HNF3.
Recently, evidence was provided that MBL2 may also be expressed in extra-hepatic tissues [17]. However, similar to our study expression was so low that the functional relevance of extra-hepatically produced MBL2 should be seriously questioned.
In spite of a promoter that resembles the promoter of acute phase genes [16], clinical investigations have revealed only minor induction of MBL2 during the acute phase response [18]. Moreover, glucocorticoids, which are acute phase agonists, reduce MBL2 gene transcription [16]. These findings are not consistent with MBL2 serving as an acute phase reactant.
We surprisingly found that every subject that received fenofibrate exhibited an increase in plasma, which means that the response was independent of genotype. Although baseline difference in plasma MBL2 are largely related to polymorphisms in the MBL2 gene, our data suggest part of the variation may be due to differences in PPARα activity and/or expression level..
Our data indicate that PPARα is unable to induce MBL2 expression in mouse liver. Previously, several genes have been reported to be specifically regulated by PPARα in human, including ApoAI, ApoAII and ApoAIV. For each of these genes the loss of regulation in mouse was related to lack of conservation of the functional PPREs. Since the PPRE(s) responsible for induction of MBL2 by PPARα remains elusive, it is impossible to determine whether a similar mechanism applies here.
MBL2 is known to be under a tight physiological regulatory system, which is evident by its stable circulating profiles in healthy individuals, independent of age, gender, time, physical exercise [21]. Alterations in MBL2 levels in disease states have been suggested to be partly caused by hormonal changes. A limited number of clinical trials have shown an stimulatory effect of growth hormone and thyroid hormones on MBL2 levels [22, 23]. The effect of growth hormone and thyroid hormone on MBL2 synthesis was reproduced in hepatocytes cell lines [24] . Our data show that plasma MBL2 levels are reduced by insulin in healthy subjects. This effect is lost in subjects with insulin resistance. The inhibitory effect of insulin on plasma MBL2 is in agreement with a previous study that reported higher MBL2 levels in patients with insulin-dependent type 1 diabetes [25], which already speculated about a suppressive effect of insulin on production of MBL2. Currently, it is unclear how our data can be reconciled with a previous cross-sectional study which showed a positive correlation between plasma MBL2 levels and insulin sensitivity [26]. Since the number of samples studied was relatively small, the suppressive effect of insulin on plasma MBL2 should be confirmed in larger studies.
In conclusion, our data point to MBL2 as potential circulating mediator of PPARα activity in human. Future studies should investigate a possible role for MBL2 in regulation of energy metabolism.

### Materials and Methods:

### Materials

Wy14643 and GW7647 was obtained from ChemSyn Laboratories (Lenexa, KS). Recombinant human insulin (Actrapid) was from Novo Nordisk (Copenhagen, Denmark). SYBR Green was from Eurogentec (Seraing, Belgium). Fetal calf serum, penicillin/streptomycin/fungizone were from Lonza Bioscience (Verviers, Belgium).
Otherwise, chemicals were from Sigma (Zwijndrecht, The Netherlands).

### Primary hepatocytes isolation

Primary human hepatocytes from 6 donors were purchased from Lonza Bioscience (Verviers, Belgium). Details of isolation and procedure are described in our previous publication (27). Briefly cells were isolated from surgical liver biopsies by two-step collagenase perfusion method and incubated in the presence or absence of Wy14643 (50 µM) dissolved in DMSO for 6 and 24 hours, followed by RNA isolation.
Mouse hepatocytes were isolated as described previously (28) from 6 different strains of mouse; NMRI, SV 129, FVB, DBA, BALB/C and CS7BL/6J.
Cells were incubated in fresh medium in the presence or absence of Wy14643 (10 µM) dissolved in DMSO for 6 and 24 hours, followed by RNA isolation. Isolation of mouse primary hepatocytes was approved by the animal ethics committee of Wageningen University.

### Affymetrix microarray

Total RNA was prepared from human and mouse primary hepatocytes using TRlzol reagent (Invitrogen, Breda, The Netherlands). RNA was used individually and further purified using RNeasy micro columns (Qiagen, Venlo, the Netherlands). To confirm RNA integrity was controlled on a bioanalyzer (Agilent Technologies, Amsterdam, the Netherlands) using 6000 Nano Chips according to the manufacturer's instructions. Five hundred nanograms of RNA were used for one cycle cRNA synthesis (Affymetrix, Santa Clara, CA). Hybridization, washing and scanning of Affymetrix Gene chip human genome U133 2.0 plus and mouse genome 430 2.0 arrays was according to standard Affymetrix protocols.
Scans of the Affymetrix arrays were processed using packages from the Bioconductor project (29). Expression levels of probe sets were calculated using GCRMA, followed by identification of differentially expressed probe sets using Limma. Genes encoding secreted proteins were selected using Gene Ontology Classification, SignalP and ngLOC (*n*-gram-based Bayesian classifier) predicting tools.

### Real time quantitative PCR

1 µg of total RNA was used for reverse-transcription with iScript (Bio-Rad, Veenendaal, the Netherlands). PCR was performed with Platinum Taq DNA polymerase (Invitrogen) on a Bio-Rad iCycler or MyIQ PCR machine. Primers were designed to generate a PCR amplification product of 100-200 bp and were taken from Primerbank (http://pga.mgh.harvard.edu/primerbank). Specificity of the amplification was verified by melt curve analysis and evaluation of efficiency of PCR amplification.. The mRNA expression reported was normalized to universal 18S gene expression.

### HepG2 Cell Culture

Human hepatoma HepG2 cells were grown in DMEM containing 10% FCS and 1% penicillin-streptomycin (20,000 units/ml potassium penicillin, 20,000µg/ml streptomycin sulfate) at 37° C/ 5 % CO2. Cells were with PPARα ligands (Wy14643 10µM, GW7647 10 µM) or vehicle (DMSO) for 6h. Cells were harvested using TRlzol (Invitrogen, Breda, The Netherlands).
For the protein measurement, HepG2 cells were incubated with GW7646 (10 µM) and Wy14643 (10 µM) for 24h. Serum was depleted before adding the ligands. The medium was collected and protein analysis was performed using commercially available MBL2 Oligomer ELISA kit.

### Chromatin immunoprecipitation (ChIP)

HepG2 cells were incubated in the absence or presence of Wy14643 (50µM) for 2 and 4h. ChIP of MBL2 transcription starting site was performed using antibodies against PPARα and POL II as previously described (30). As control, antibody against immunoglobulin G (IgG) was used. Sequences of primers used for PCR were 5'-GGCATCCTCACAGGTCACAG-3' for the forward primer and 5'-CGGTCCCATTTGTTCTCACTG-3' for the reverse primer.

### Human subjects

In fenofibrate study fasted blood samples were taken from 19 subjects between 30-70 y of age with metabolic syndrome before and after a 6-week treatment with fenofibrate (200 mg of micronized daily). Samples were kind gift of Dr. Ronald P. Mensink. In fasting study, blood was taken from 3 healthy male between 19 and 22 y of age who were recruited from the Wageningen student population. The samples were from a published study (31). Briefly volunteers received an identical meal at 1700, before the start of a 48-h fasting period. During the fasting period, the subjects were not allowed to eat or drink anything except water. After 48 h, the volunteers received a light meal. At baseline and 48 h of fasting, blood samples were taken. All human experiments were approved by the medial ethics committee of Wageningen University and Maastricht University and subjects were informed about the design and purpose of the study and provided full informed written consent.

### Plasma analysis

Plasma MBL2 levels were determined using a commercially available MBL2 Oligomer Elisa kit (ITK diagnostics BV, Uithoorn, the Netherlands) using biotinylated monoclonal detection antibody following manufacturers instructions.

### Hyperinsulinemic-isoglycemic clamp

Six healthy subjects and 9 T2DM patients under went a 180-minute hyperinsulinemic-isoglycemic clamp (32). Samples were from a published study (ref).
In brief, two cannulas were inserted into forearm veins for infusion and sampling. Insulin was infused (20 IU HM-R insulin in 20 ml 0.9 % saline solution, infusion rate: 1 mIU/kg/min for patients with BMI <30 kg/m2, 40 mIU/m2/min for patients with BMI >30 kg/m2) and glucose solution (20 % glucose solution) + 20 ml 7.5 % KCI in 500 ml 0.9 % sodium saline solution. Glucose infusion rate was modified according to changes of blood glucose concentration. Blood glucose was measured every 5 minutes and insulin measurements was done at baseline and steady-state period. Blood glucose concentration was measured by glucometer (Super Glucocard II, Arkray, Japan).
Glucose clamp was finished when glucose level was close or equal to fasting blood glucose level for at least 30 minutes.

### Statistical analysis

Statistical significant differences were calculated using Student's T-test. The cut-off for statistical significance was set at a P-value of 0.05 or below. The non normalized data were log transformed.

### References

1. Nishimura, T., et al., Identification of a novel FGF, FGF-21, preferentially expressed in the liver. Biochim Biophys Acta, 2000. 1492(1): p. 203-6.
2. Kersten, S., et al., Peroxisome proliferator-activated receptor alpha mediates the adaptive response to fasting. J Clin Invest, 1999. 103(11): p. 1489-98.
3. Leone, T.C., C.J. Weinheimer, and D.P. Kelly, A critical role for the peroxisome proliferator-activated receptor alpha (PPARalpha) in the cellular fasting response: the PPARalpha-null mouse as a model of fatty acid oxidation disorders. Proc Natl Acad Sci U S A, 1999. 96(13): p. 7473-8.
4. Kersten, S., et al., Characterization of the fasting-induced adipose factor FIAF, a novel peroxisome proliferator-activated receptor target gene. J Biol Chem, 2000. 275(37): p. 28488-93.
5. Lundasen, T., et al., PPARalpha is a key regulator of hepatic FGF21. Biochem Biophys Res Commun, 2007. 360(2): p. 437-40.
6. Inagaki, T., et al., Endocrine regulation of the fasting response by PPARalpha-mediated induction of fibroblast growth factor 21. Cell Metab, 2007. 5(6): p. 415-25.
7. Badman, M.K., et al., Hepatic fibroblast growth factor 21 is regulated by PPARalpha and is a key mediator of hepatic lipid metabolism in ketotic states. Cell Metab, 2007. 5(6): p. 426-37.
8. Kersten, S., Regulation of lipid metabolism via angiopoietin-like proteins. Biochem Soc Trans, 2005. 33(Pt 5): p. 1059-62.
9. Heinaniemi, M., et al., Meta-analysis of primary target genes of peroxisome proliferator-activated receptors. Genome Biol, 2007. 8(7): p. R147.
10. Lefterova, M.I., et al., PPARgamma and C/EBP factors orchestrate adipocyte biology via adjacent binding on a genome-wide scale. Genes Dev, 2008. 22(21): p. 2941-52.
11. Nielsen, R., et al., Genome-wide profiling of PPARgamma: RXR and RNA polymerase II occupancy reveals temporal activation of distinct metabolic pathways and changes in RXR dimer composition during adipogenesis. Genes Dev, 2008. 22(21): p. 2953-67.
12. Bouwens, M., L.A. Afman, and M. Muller, Fasting induces changes in peripheral blood mononuclear cell gene expression profiles related to increases in fatty acid beta-oxidation: functional role of peroxisome proliferator activated receptor alpha in human peripheral blood mononuclear cells. Am J Clin Nutr, 2007. 86(5): p. 1515-23.
13. Thiel, S., et al., A second serine protease associated with mannan-binding lectin that activates complement. Nature, 1997. 386(6624): p. 506-10.
14. Stienstra, R., et al., PPARs, Obesity, and Inflammation. PPAR Res, 2007. 2007: p. 95974.
15. Steffensen, R., et al., Detection of structural gene mutations and promoter polymorphisms in the mannan-binding lectin (MBL) gene by polymerase chain reaction with sequence-specific primers. J Immunol Methods, 2000. 241(1-2): p. 33-42.
16. Naito, H., et al., Characterization of human serum mannan-binding protein promoter. J Biochem, 1999. 126(6): p. 1004-12.
17. Seyfarth, J., P. Garred, and H.O. Madsen, Extra-hepatic transcription of the human mannose-binding lectin gene (mbl2) and the MBL-associated serine protease 1-3 genes. Mol Immunol, 2006. 43(7): p. 962-71.
18. Thiel, S., et al., The concentration of the C-type lectin, mannan-binding protein, in human plasma increases during an acute phase response. Clin Exp Immunol, 1992. 90(1): p. 31-5.
19. Christodoulides, C., et al., Circulating FGF21 is induced by PPAR agonists but not ketosis in Man. J Clin Endocrinol Metab, 2009.
20. Galman, C., et al., The circulating metabolic regulator FGF21 is induced by prolonged fasting and PPARalpha activation in man. Cell Metab, 2008. 8(2): p. 169-74.
21. Ytting, H., et al., Biological variation in circulating levels of mannan-binding lectin (MBL) and MBL-associated serine protease-2 and the influence of age, gender and physical exercise. Scand J Immunol, 2007. 66(4): p. 458-64.
22. Hansen, T.K., et al., GH strongly affects serum concentrations of mannan-binding lectin: evidence for a new IGF-I independent immunomodulatory effect of GH. J Clin Endocrinol Metab, 2001. 86(11): p. 5383-8.
23. Riis, A.L., et al., Thyroid hormone increases mannan-binding lectin levels. Eur J Endocrinol, 2005. 153(5): p. 643-9.
24. Sorensen, C.M., et al., Hormonal regulation of mannan-binding lectin synthesis in hepatocytes. Clin Exp Immunol, 2006. 145(1): p. 173-82.
25. Hansen, T.K., et al., Elevated levels of mannan-binding lectin in patients with type 1 diabetes. J Clin Endocrinol Metab, 2003. 88(10): p. 4857-61.
26. Fernandez-Real, J.M., et al., Protection from inflammatory disease in insulin resistance: the role of mannan-binding lectin. Diabetologia, 2006. 49(10): p. 2402-11.
27. Rakhshandehroo, M., et al., Comparative analysis of gene regulation by the transcription factor PPARalpha between mouse and human. PLoS One,2009. 27;4(8):e6796
28. Kuipers F, Jong MC, Lin Y, Eck M, Havinga R, Bloks V, et al. Impaired secretion of very low density lipoprotein-triglycerides by apolipoprotein E-deficient mouse hepatocytes. J Clin Invest, 1997.100:2915-2922.
29. Gentleman, R.C., et al., Bioconductor: open software development for computational biology and bioinformatics. Genome Biol. 2004;5:R80.
30. Degenhardt, T., et al., Peroxisome proliferator-activated receptor alpha controls hepatic heme biosynthesis through ALAS1. J Mol Biol, 2009. 1;388(2):225-38.
31. Bouwens, M., et al., Fasting induces changes in peripheral blood mononuclear cell gene expression profiles related to increases in fatty acid beta-oxidation: functional role of peroxisome proliferator activated receptor alpha in human peripheral blood mononuclear cells. Am J Clin Nutr, 2007. 86(5):1515-23.
32. Phielix E. et al, Diabetes, 2008. 57: 2943-2949.

## Claims

1. Use of a MBL2 as a biomarker of liver-specific insulin resistance.

2. A method for diagnosing the presence of liver-specific insulin resistance in a subject, the method comprising the steps of:
(a) determining the expression level of a MBL2 gene represented by a nucleic acid sequence having at least 60% identity with SEQ ID NO: 1 in a subject; and,
(b) comparing the expression level of said gene as defined in (a) with a reference value for said expression level, preferably wherein the reference value is the average value for said expression level in a control subject.

3. A method according to claim 2, wherein a liver-specific insulin resistance is diagnosed when the comparison leads to the finding of a detectable expression level or an increase of the expression level of said gene.

4. A use or a method according to any one of claims 1 to 3, wherein the presence of MBL2 is determined by directly quantifying the amount of encoded polypeptide and/or indirectly by quantifying the amount of a corresponding nucleotide molecule.

5. A use or a method according to any one of claims 1 to 4, wherein the presence of MBL2 is determined *ex vivo* in a sample obtained from the subject.

6. A use or a method according to claim 5, wherein the sample is a fluid obtained from the subject, preferably wherein the fluid is selected from: plasma, faeces, urine, blood, or saliva, more preferably wherein the fluid comprises or consists of plasma.

7. A use or a method according to claim 5, wherein the presence of a MBL2 polypeptide is assessed in the plasma of a single subject before and after hyperinsulinemic clamp, preferably for diagnosing a liver-specific insulin resistance.

8. A use or a method according to any one of claims 1 to 6, wherein the MBL2 gene is represented by SEQ ID NO: 1.

9. A use or a method according to any one of claim 1 to 7, wherein MBL2 is the single biomarker used, preferably for diagnosing a liver-specific insulin resistance.

10. A method for identification of a substance capable of preventing, treating and/or delaying the progression of liver-specific insulin resistance in a subject, the method comprising the steps of:
(a) providing a test cell population or a test animal capable of expressing a gene represented by a sequence as identified in claim 2;
(b) contacting the test cell population or the test animal with the substance;
(c) determining the expression level of the gene encoding a polypeptide or the activity or steady state level of the encoded polypeptide in the test cell population or in the test animal contacted with the substance;
(d) comparing the expression, activity or steady state level determined in (c) with the expression, activity or steady state level of the gene or of the polypeptide in a test cell population or in a test animal that is not contacted with the substance; and,
(e) identifying a substance that produces a difference in expression level, activity or steady state level of the gene or the polypeptide, between the test cell population or test animal that is contacted with the substance and the test cell population or test animal that is not contacted with the substance.

11. A method according to claim 9, whereby the difference identified in step (d) produced by the substance is a decrease of the expression level of said gene.
